# EUROPEAN PATENT SPECIFICATION

(11) **EP 3 238 611 B1**
(45) Date of publication and mention of the grant of the patent: **17.11.2021**
(21) Application number: 16167619.2
(22) Date of filing: 29.04.2016
(51) Int. Cl.: A61B 5/00, A61B 5/16

(54) **A METHOD AND DEVICE FOR ESTIMATING A CONDITION OF A PERSON**
VERFAHREN UND VORRICHTUNG ZUR BEURTEILUNG DES ZUSTANDS EINER PERSON
PROCÉDÉ ET DISPOSITIF PERMETTANT D'ESTIMER L'ÉTAT D'UNE PERSONNE

(43) Date of publication of application: 01.11.2017
(73) Proprietor: Stichting IMEC Nederland, 5656 AE Eindhoven (NL)
(72) Inventor: CASALE, Pierluigi, 3001 Leuven (BE); LAMICHHANE, Bishal, 3001 Leuven (BE); GROSSEKATHOEFER, Ulf, 3001 Leuven (BE)
(74) Representative: AWA Sweden AB

(56) References cited:
- WO-A2-2012/100081
- US-A1- 2015 157 273
- US-A1- 2016 078 771
- SMETS ET AL: "Comparison of Machine Learning Techniques for Psychophysiological Stress Detection", 1 January 2016 (2016-01-01), PERVASIVE COMPUTING PARADIGMS FOR MENTAL HEALTH, SPRINGER, CH, PAGE(S) 13 - 22, XP008182014, ISBN: 978-3-319-32269-8 * abstract *
- MARTON ZOLTAN-CSABA ET AL: "Ensembles of strong learners for multi-cue classification", PATTERN RECOGNITION LETTERS, ELSEVIER, AMSTERDAM, NL, vol. 34, no. 7, 27 July 2012 (2012-07-27), pages 754-761, XP029002985, ISSN: 0167-8655, DOI: 10.1016/J.PATREC.2012.07.011

## Description

### Technical Field

The present invention relates to a method and device for estimating a condition of a person. In particular, the present invention relates to estimating of a stress level of a human.

### Background

There is growing worldwide awareness of the problems caused by long-term stress, such as depression, burn-out and cardiovascular disorders. The number of workdays lost due to anxiety, stress and neurotic disorders is four times higher than the number of lost days due to other non-fatal injuries and illnesses. In the European Union, more than 40 million individuals are affected by work-related stress. Stress is one of the most commonly reported causes of occupational illness by workers and costs approximately 20 billion euros per year in lost productivity and medical expenses, as reported in Institute of Work, Health & Organisations, "Towards the development of a European framework for psychosocial risk management at the workplace", 2008. Stress comes in many flavors and has many aspects. Biological stress describes the physiological reaction to stressors or threads. Psychological stress is related to psychological, social reactions and consequences caused by stress. Stress can lead to diseases that influence the ability to work, and affects the social environment such as the families of stressed persons. Biologically, stress is a strategy to address threatening situations and events in order to increase survival chances. For example, if an animal is confronted with an attack of a predator, stress triggers decision processes outside the usual channels, allows quicker reactions and, thus, increases the chances of survival.

Stress leads to various physiological responses in humans including increased adrenaline and cortisol levels, increased heart rate, dry mouth, dilated pupils, bladder relaxation, tunnel vision, shaking, flushed face, slowed digestion, hearing loss, and change in the electrical properties of the skin.

Stress and its physiological manifestation that can be measured by biosensors is an utmost personal phenomenon. Each individual reacts differently to stress, and thus, the readings from biosensors are different. For example, for one individual, stress might manifest itself in a permanently increased heart rate, whereas another individual might show variation in electrodermal response. Such a wide variety of possible physiological responses makes it difficult to develop a generalized stress estimator that can predict the stress level of all persons. Instead, it is required to tailor the estimator to each individual.

Further, none of those physiological responses are exclusively caused by stress. Instead, there are various reasons that alter physiological signals in a similar way as stress does and other events or situations not relating to stress can yield physiological responses similar to stress. For example, the heart rate increases also if a person performs physical demanding activities, and the electrodermal properties of the skin are influenced by temperature and humidity of the surrounding air.

EP 2 845 539 A1 discloses a method for calculating normalized physiological signals of a living being. The method takes in consideration differences in physiology signals due to environmental factors, for example, by using a different model based on location information of the living being, and thus reducing differences within individuals due to environmental conditions. The method is capable of calculating a stress level from the normalized physiological signals.

However, combining multiple sensors to a multimodal sensor stream is prone to errors. For example, sensors might fail without notice due to hardware failures, or particular setups allow to only measure a subset of bio-signals that are present in the stress estimator. It would be desired to have a robust stress estimator, that is able to address those issues without retraining and cost-intensive data collection and labeling. US 2015/0157273 A1 discloses a device to detect heart failure based on a classifier fusion technique. Other relevant prior art is disclosed in WO 2012/100081 A2, and in "Ensembles of strong learners for multi-cue classification", Marton et al., Pattern Recognition Letters 34 (2013) pp. 754-761.

### Summary of the Invention

It is an object of the invention to provide a method and device for estimating a condition of a person, which is robust and which is possible to adapt to an individual. It is further object to provide a method and device which incorporates contextual information.

These and other objects of the invention are at least partly met by the invention as defined in the independent claims. Preferred embodiments are set out in the dependent claims.

According to a first aspect of the invention, there is provided a computer-implemented method for estimating a condition of a person, said method comprising: receiving a set of measurement values, said set of measurement values comprising a plurality of physiological measurement values applying to the condition of the person and a plurality of context measurement values, wherein said set of measurement values are obtained by a set of sensors comprising a plurality of physiological sensors and at least one environment sensor, wherein each physiological measurement value is obtained by a physiological sensor making a measurement on the person and wherein each context measurement value is obtained by an environment sensor making a measurement on an environment in which the person is located; inputting the set of measurement values to an ensemble of learners for estimating the condition of the person, wherein each learner in the ensemble of learners is trained to make an estimate of the condition of the person based exclusively on features which are extracted from measurements by a single physiological sensor or environment sensor and wherein each learner, depending on availability amongst the received set of measurement values, receives measurement values relevant to the features as input; computing, by a learner in the ensemble of learners for which measurement values corresponding to the features used by the learner is available, an individual estimate value of the condition of the person, wherein said computing is performed by a plurality of learners to determine a plurality of individual estimate values; receiving weights to be applied to the individual estimate values determined by the learners in the ensemble of learners, wherein the weights are at least partly adapted to individual characteristics of the person; and combining individual estimate values based on the received weights to make a final estimate of the condition of the person.

According to a second aspect of the invention, there is provided a computer program product comprising a computer-readable medium with computer-readable instructions such that when executed on a processing unit the computer program product will cause the processing unit to perform the method according to the first aspect.

According to a third aspect of the invention, there is provided a device for estimating a condition of a person, said device comprising: a data input module, which is configured to receive a set of measurement values, said set of measurement values comprising a plurality of physiological measurement values applying to the condition of the person and a plurality of context measurement values, wherein said set of measurement values are obtained by a set of sensors comprising a plurality of physiological sensors and a plurality of environment sensors, wherein each physiological measurement value is obtained by a physiological sensor making a measurement on the person and wherein each context measurement value is obtained by an environment sensor making a measurement on an environment in which the person is located; a trained machine learning module, which comprises an ensemble of learners for estimating the condition of the person, wherein each learner in the ensemble of learners is trained to make an estimate of the condition of the person based exclusively on features which are extracted from measurements by a single physiological sensor or environment sensor, wherein said trained machine learning module is configured to receive the set of measurement values from the data input module and wherein each learner, depending on availability amongst the received set of measurement values, receives measurement values relevant to the features as input; said trained machine learning module being further configured to compute, by a learner in the ensemble of learners for which measurement values corresponding to the features used by the learner is available, an individual estimate value of the condition of the person, wherein said computing is performed by a plurality of learners to determine a plurality of individual estimate values; and a combiner module, which is configured to receive weights to be applied to the individual estimate values determined by the learners in the ensemble of learners, wherein the weights are at least partly adapted to individual characteristics of the person; and wherein the combiner module is further configured to combine individual estimate values based on the received weights to make a final estimate of the condition of the person.

Thanks to the invention, each learner in an ensemble of learners for estimating the condition is trained to make an estimate of the condition based exclusively on features which are extracted from measurements by a single physiological sensor or environment sensor. This implies that if a sensor fails or is not available, learners which use input from other sensors are not affected at all. Hence, the estimation of the condition of a person may be very robust, as there is a possibility to fully use all learners that take input from the sensors that still function and are available.

Further, a relevance of measurements by a sensor may differ substantially for different persons, e.g. when a stress level is to be estimated. Thanks to learners being based exclusively on featurese extracted from measurements by a single sensor, the combining of individual estimate values from different learners may be easily personalized. Thus, the weight to be given to a learner being trained on measurements from a specific sensor may be adapted to individual characteristics reflecting the relevants of these measurements.

An ensemble of learners is normally used in machine learning to obtain better predictive performance than could be obtained from any of constituent learning algorithms. According to the present invention, each learner in the ensemble is trained on a single sensor modality, such that there is robustness to the possibility of making an estimate of the condition of a person.

It should be realized that the individual estimate values computed by learners in the ensemble need not be performed by all learners for which relevant measurement values are available. For a specific individual, the learner may not be relevant, even though measurement values are available. Then, instead of computing the individual estimate value and applying a zero weight to the individual estimate value, the individual estimate value need not be computed at all by such learner.

The learners may be weak learners, which in combination in the ensemble are able to make a reliable estimate of the condition of the person.

According to an embodiment, the weights are at least partly based on a personal dataset for the person whose condition is to be estimated, the personal dataset relating measurement values obtained for the person to levels of the condition. Thus, the final estimate established by combined individual estimate values may be trained in relation to a personal dataset for a specific individual, such that the weights may be optimized for reliably estimating the condition for the individual.

According to another embodiment, the weights are at least partly based on a database storing information about weights in relation to characteristics of persons. For instance, anthropometric features, such as gender, age, body mass index, behaviour, may be collected during training of the ensemble of learners such that optimal weights in relation to the anthropometric features may be determined and stored in a database. Thus, the weights may be selected from the database such that, even if no training is performed for a specific individual, weights that are likely to be relevant for the individual may be used.

According to an embodiment, the method further comprises selecting learners that are relevant for estimating the condition for the person, wherein said computing is performed by the selected learners. Using relevance of learners for an individual, the learners that are relevant may be selected, such that individual estimate values need only be computed for the selected learners.

According to an embodiment, the method further comprises detecting that no measurement values are received from a sensor among the set of sensors; and disregarding learners among the ensemble of learners that are trained to make an estimate of the condition of the person based on features which are extracted from measurements by the sensor from which no measurement values are received.

In case of failure of a sensor or that a sensor is not available, some learners will not receive measurement to be able to make individual estimates of the condition. Therefore, these learners may be disregarded so as not to interfere with the final estimate of the condition. Further, thanks to each learner being trained based on features extracted from measurements by a single sensor, there will be other learners available so as to enable a final estimate of the condition to be determined.

According to an embodiment, the plurality of context measurement values in the set of measurement values are inputted to a weight selector for determining weights, wherein the weights are at least partly adapted to the environment in which the person is located. Hence, the weights to be used for individual estimate values may be adapted to a context in which the measurements are made. Thus, different weights may be given to individual estimate values depending on the context. For example, lesser weight may be give to an individual estimate value based on increased heart rate, if it simultaneously detected that the person is at a gym.

According to an embodiment, the method further comprises:
determining a number of learners which are to contribute to a final estimate of the condition of the person; and adapting the received weights based at least on the determined number of learners for which individual estimate values are to contribute to the final prediction value. Thus, the method may re-normalize the weights to be used in dependence of how many learners are to contribute to the final estimate. The weights may initially be set in relation to using a combination based on individual estimate values from all learners. When some learners will no longer provide individual estimate values (because a sensor fails or is not available), the weights may be adapted to the actual learners to be used.

According to an embodiment, the condition is a stress level of the person. The method is especially suited for estimating a stress level, since many different sensor modalities may contribute to making a good prediction of the stress level and it may therefore be of interest to have a robust method which is able to reliably determine a stress level even if some sensor fails or is not available.

It should be realized that other conditions may be estimated alternatively or additionally to estimation of a stress level, wherein a plurality of sensors are used for obtaining data relevant for estimating the condition and, thus, there is a benefit of having robustness with regard to sensor failure or unavailability. For instance, a psychological state of the person may be estimated.

As another alternative, the method may be used to estimate or monitor pain experienced by a person. This could be used, for instance, for a person recovering from a surgical operation, wherein sensor(s) may be arranged to be wearable, in order to detect and monitor pain of the person.

According to an embodiment, the set of measurement values comprises at least one in the group of: physiological measurement values from an electrocardiogram, ECG, sensor; physiological measurement values from a galvanic skin response, GSR, sensor; context measurement values from a light level sensor; context measurement values from a positioning sensor. These types of measurement values may be relevant for estimating a stress level of a person. The ECG and GSR sensors may obtain physiological values which may be correlated to a stress level. Further, a position of the person may indicate a context which affects how the physiological measurement values are to be interpreted. For instance, if the person is e.g. at home, at work or at the gym, the physiological measurement values corresponding to an experienced stress may differ. Similarly, a light level in the environment may also affect the physiological measurement values such that the estimation of stress level need to be adapted to the measured light level.

### Brief Description of Drawings

The above, as well as additional objects, features and advantages of the present inventive concept, will be better understood through the following illustrative and non-limiting detailed description of preferred embodiments of the present invention, with reference to the appended drawings. In the drawings like reference numerals will be used for like elements unless stated otherwise.
Fig. 1 is a schematic view of a device for estimating a condition of a person according to an embodiment.
Fig. 2 is a schematic view of a relation between learners and sensors within the device.
Fig. 3 is a schematic view of a connection between a combiner module and databases for providing weight information.
Fig. 4 is a graph illustrating results from a study comparing classification accuracies for different weight configurations.
Fig. 5 is a flow chart of a method according to an embodiment.

### Detailed Description

Detailed embodiments of the present invention will now be described with reference to the drawings.

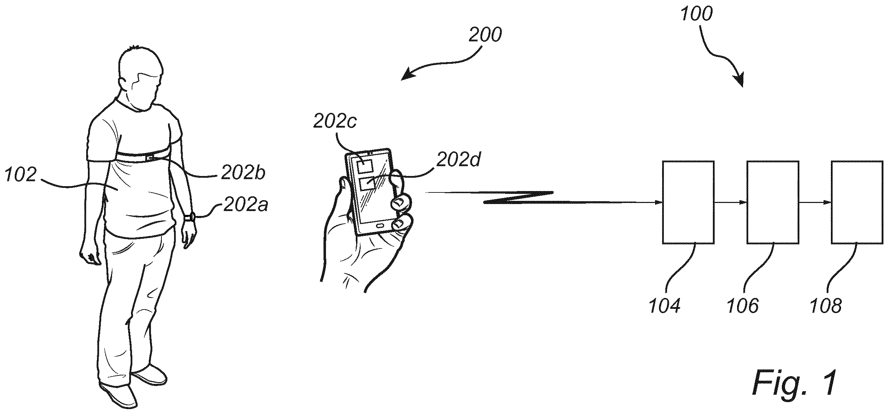

Referring to Fig. 1, a device 100 for estimating a condition of a person 102 will first be described. The device 100 may be arranged within a system 200, in which measurements for collecting data in order to determine an estimate of the condition are performed.

The system 200 may comprise a plurality of sensors 202a-d, including a plurality of physiological sensors 202a-b and a plurality of environment sensors 202c-d.

The physiological sensors 202a-b may be sensors which are arranged to obtain physiological measurement values. The physiological sensor 202a-b may, for instance, be arranged to acquire an electrocardiogram, ECG, or a galvanic skin response, GSR. The physiological sensor 202a-b may alternatively or additionally be arranged to acquire a simple heart rate (without acquiring an ECG), an electroencephalogram (EEG), a bioimpedance, etc.

The physiological sensor 202a-b may be arranged as a wearable and may be continuously worn by the person 102. For instance, the physiological sensor 202a-b may be arranged in a garment, or may be arranged in a wearable device, such as a watch or a strap which is worn around the chest to measure heart information.

A person 102 may be wearing such physiological sensors 202a-b anyway, as the physiological sensors 202a-b may e.g. be part of a smart watch.

The environment sensors 202c-d may be any sensors which are arranged to obtain measurements to provide a context of the person 102. The environment sensors 202c-d may, for instance, be arranged to acquire a position of the person, lighting conditions in the environment, a speed of movement of the person, etc.

The environment sensors 202c-d may also be arranged as a wearable and may be continuously worn by the person 102. The environment sensors 202c-d may alternatively be arranged in a portable device, such as a mobile phone, which the person 102 may be carrying most of the time. For instance, the environment sensors 202c-d may be a Global Positioning System (GPS) sensor, a light sensor, and/or an accelerometer, which may all be arranged in the mobile phone.

The physiological sensors 202a-b may be connected through wire or wirelessly to the device 100 for providing measurement values to the device 100. Similarly, the environment sensors 202c-d may be connected through wire or wirelessly to the device 100 for providing measurement values to the device 100.

According to an alternative, the physiological sensors 202a-b and/or the environment sensors 202c-d may be arranged within a common housing with the device 100. For instance, a mobile phone may be provided with software, such as an application, for performing computations so as to estimate the condition of the person 102.

The physiological sensors 202a-b and/or the environment sensors 202c-d may alternatively have a wired or wireless connection to an intermediate device and may provide measurement values to the intermediate device. The intermediate device may forward the measurement values to the device 100, such that the device 100 may be arranged in a server, which receives information from the intermediate device e.g. over a computer network, such as the Internet.

The device 100 may be implemented in hardware, or as any combination of software and hardware. The device 100 may, for instance, be implemented as software being executed on a general-purpose computer, as firmware arranged e.g. in an embedded system, or as a specifically designed processing unit, such as an Application-Specific Integrated Circuit (ASIC) or a Field-Programmable Gate Array (FPGA). In a specific embodiment, the device 100 is arranged in a processing unit of a mobile phone, which is provided with a computer program for controlling the processing unit to perform a process for estimating a condition of the person 102.

The device 100 comprises a data input module 104, which is configured to receive a set of measurement values from the physiological sensors 202a-b and the environment sensors 202c-d.

The device 100 further comprises a trained machine learning module 106, which is trained for estimating a condition of the person 102 based on measurement values, as will be further described below. The trained machine learning module 106 may comprise an ensemble of learners, and the learners may compute individual estimate values of the condition.

The device 100 further comprises a combiner module 108, which is configured to receive weights to be applied to the individual estimate values and to comine the individual estimate values based on the received weights to make a final estimate of the condition.

The device 100 is able to continuously and automatically update and recalculate the condition of a person 102 over time by gathering updated data from physiological measurements and updated information about the context the person 102 is in.

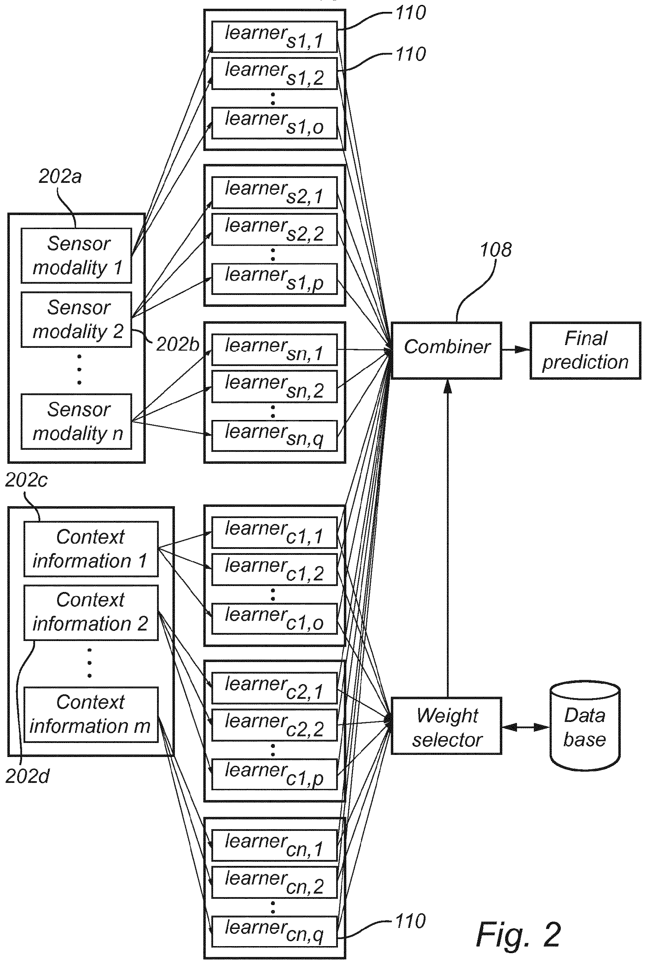

Referring now to Fig. 2, the estimation of a condition by the device 100 will be described in more detail. In the described device 100, a learner 110 is a trained machine learning model. Such machine learning model could be, for example, a random forest, support vector machine, decision tree etc. The learners 110 are trained with a set of features all derived from a single sensor modality. Each learner 110 is trained on different subset of features for one single sensor or context modality, as indicated in Fig. 2, where each learner 110 receives input from only a single sensor 202a-d.

The working of estimation of a condition of the person 102 is demonstrated with one representative example, wherein a stress level of the person 102 is to be estimated. Consider a system consisting of an ECG and a GSR sensor 202a-b measuring the physiology, and an ambient light sensor 202c and a microphone 202d measuring the context information.

A possible ECG feature set could be:
*i. Mean heart rate (mhr)*
*ii. Standard deviation of R-R peak intervals (sdnn)*
iii. *Root mean of sum of squared difference of consecutive R-R peak intervals (rmssd)*
*iv. Low frequency component of the spectrum* of *R-R peak intervals (LF): power in the 0.04 Hz-0.15 Hz band*
v. *High frequency component of the spectrum* of *R-R peak intervals (HF): power in the 0.15 Hz-0.4 Hz band*
vi. *Ratio of LF to HF (LFHF)*
vii. *Percentage of R-R peak intervals that are greater than 50 milliseconds (pnn50)*
viii. *Approximate entropy of R-R peak intervals (apen)*
ix. *Length of the major axis of poincare plot (SD1)*
x. *Length off minor axis of poincare plot (SD2)*
xi. *Ratio of the axes of poincare plot (SD1*/*SD2)*

A possible GSR feature set is:
*i. Skin conductance level (scl)*
ii. *Signal power of the skin conductance signal (scp)*
iii. *Skin conductance response rate (scrr)*
iv. *Signal power in the second order difference of the skin conductance signal (scdiff2).*

An example of feature set from the measured sound signal through microphone is:
i. *Sound level (slevel)*
ii. *Dominant Frequency (Dfreq)*
iii. *Cepstral Coefficients (Ccoeff)*
iv. *Pitch-related features (Pfeat) e.g. pitch period and harmonics-to-noise ratio*
v. *Energy-related features (Efeat)*
vi. *Linear predictor coefficients (LPC)*

An exemplary feature set computed from the measured ambient light is:
i. *Mean light level (mlight)*
ii. *Variability of the light level (vlight)*
iii. *Median light level (medlight)*
iv. *Range of ambient light level (rangelight)*

An example of learners 110 are the machine learning algorithm trained on following feature subsets: [{mhr, sdnn, rmssd}, {LF, HF, LFHF, apen}, {mhr, SD1, SD2, SD1SD2},{rmssd, LF}, {scl, scp}, {scdiff2, scl, scp}, {Slevel, Dfreq, Ccoeff},{Ccoeff, Pfeat, Efeat}, {LPC, Pfeat, Efeat}, {mlight, vlight, medlight}, {rangelight, medlight, mlight}].

It is to be noted that each feature subset is strictly derived from a single sensor modality 202a-d. The described feature subset is just a representative example. One possible method to generate the feature subset from the available feature set is bootstrapping. Each of the learners 110, specializing in a feature subset for a modality, is capable to give decision about the corresponding stress level against which the learners 110 have been trained. The learner 110 could be a classifier, which is able to predict for different discrete stress level (stress states) or a regressor, which is able to provide the prediction of stress level on a continuous scale.

The combiner module 108 produces a final decision on the stress level by combining the stress level reported by each of these learners 110. The combiner module 108 receives as input the estimated stress level from individual learners 110 on both physiological and contextual signal modalities. Within the combiner module 108 each of these decision channels are weighted and combined to produce a final decision.

A number of factors are considered in assigning the weights for these individual decision channels, which allows for personalization and contextualization of the estimation of the condition.

The combiner module 108 may receive information about anthropometric features of the person 102. Some examples of such anthropometric features are age, weight, behavior etc. These anthropometric features are cross-referenced with a database of information consisting of optimal weights computed for (a group of) individuals with their corresponding anthropometric features. The optimal weight for the person 102 in test is chosen according to the weights of the person/group in the database who is closest to the person 102 in test in the anthropometric feature space.

The combiner module 108 also has access to the information about the context for the person in test. This could for example be the location e.g. work, house, shopping market. The combiner module 108 may thus take the context into account and an optimality of the weights is dependent on the context. The database may thus have a further fine-grained detail about the optimal weights for various high level contexts.

The combiner module 108 may thus turn the final estimation of the condition into both personalized and contextualized estimation.

Further, the estimation is robust of sensor failures or differing sensor configuration. The system 200 for the person 102 in test could consist of only a subset of information signal modalities as compared to the information signal modality that was used for training the device 100. In such case, the device 100 is still able to produce an estimate of the condition. The learners 110 corresponding to a missing/faulty sensor modality is not able to produce any individual estimate values. However, the combiner module 108 may be aware of this and re-normalizes the weight configured for other decision channels.

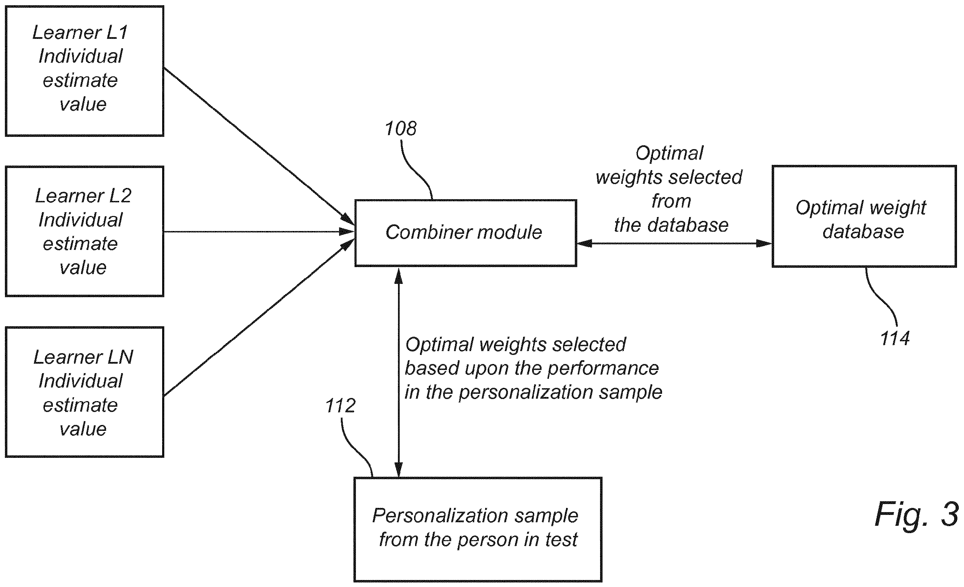

As illustrated in Fig. 3, the device 100 may be arranged in two different ways to enable personalization depending upon the availability of a personalization sample. Personalization sample is a small set of labeled dataset for the person 102 in test.

First, in the presence of personalization sample, personalization works by learning the optimal weights, which gives good performance in the personalization sample. The combiner module 108 may thus retrieve weights to be used from a database 112, which stores weights based on the personalization sample.

Second, in the absence of personalization sample, optimal weights are retrieved from the database 114 where the information about optimal weights for different (group of) person with a given anthropometric feature is stored.

A hybrid approach, in the presence of personalization sample, is also possible where the weights fit for the personalization sample in database 112 is fused with the weights drawn from the optimal weight database 114.

Stress response has a context factor. Stressors experienced at the workplace are different than the stressors present at home. There might be varying responses from a same individual based upon the context the person 102 in test is. Similarly different individuals might show higher similarity of stress response in similar context. For example, people working in same office environment are generally exposed to same kind of stressors. The combiner module 108 allows the possibility to take the context factors to stress response into account. This is embedded together with the personalization function.

Where personalization samples are available, the contextual factor may be included by optimizing the weights only on the subset of personalization samples specific or very close of a particular context. The weights are adapted with the personalization sample as the changing context of the person 102 in test is identified.

Where personalization samples are not available, the contextual factor is included based upon the pre-defined information available in the optimal weights database 114. The optimal weight database 114 has the optimal weights definition attached to the context within which it was derived.

In case a sensor or a sensor modality 202a-d fails or is not available due to, for example sensor failure or user behavior, the device 100 is able to provide a meaningful output based on the remaining sensors / sensor modalities 202a-d.

The individual estimate values of the remaining learners 110 reach the combiner module 108, where these values are fused while ignoring the missing values. This could be, for example, by dynamically setting the weight values of the learners 110 associated with missing modalities to zero, or by simply skipping over these individual estimate values in the combination process. In order to provide an identically distributed final output prediction from the combiner module 108, it might be necessary to re-normalize the weights that are associated with the single learners' estimates to a defined value, for example, one. The normalization will adjust the weights for the remaining learners 110 by the number of available learner predictions.

An example of pseudo code for weight adjustment to allow robust combination towards missing individual estimates of learners 110 is as follows:

```
       normalization_factor := 0
       for weight_learner_i in {available weights}:
             normalization_factor := normalization_factor + weight_learner_i
       for weight_learner_i in {available weights}:
             weight_learner_i := weight_learner_i /normalization_factor
```

An example of pseudo code for a combination operation in the combiner module 108 is as follows:

```
       prediction_value := 0
       for i in 1:num_learners:
             prediction_value := weight_learner_i*predicition_learner_i
       final_predicition := prediction_value / num_learners
```

An example of pseudo code for a combination operation in the combiner module 108 that only uses estimate values from available learners 110 is as follows:

```
       prediction_value := 0
       num_valid_learners := 0
       for i in 1:num_learners:
             if prediction_learner_i is available:
                    prediction_value := weight_learner_i*predicition_learner_i
                    num_valid_learners := num_valid_learners + 1
       final_predicition := prediction_value / num_valid_learners
```

An experiment has been performed to investigate if incorporating information about different group of people with a given anthropometric feature can be used to increase the estimation accuracy of self-reported stress levels. In order to answer this research question, random forest classifiers (learners) were evaluated for
(a) Data collected from a set of participants without incorporating anthropometric information (general learner).
(b) Data only for a subset of participants that share a common anthropometric feature. For demonstration purpose, the data from all female participants available in the study corpus (anthropometric learner) was selected.

These learners are then combined in an ensemble as suggested above. The weights were manually defined to
(a) General configuration: w_general = 1, w_anthropometric = 0.
(b) Anthropometric configuration: w_general = 0, w_anthorpometric = 1.

An out-of-bag error validation scheme was incorporated where data that is not used in training one tree from the random forest is used for testing.

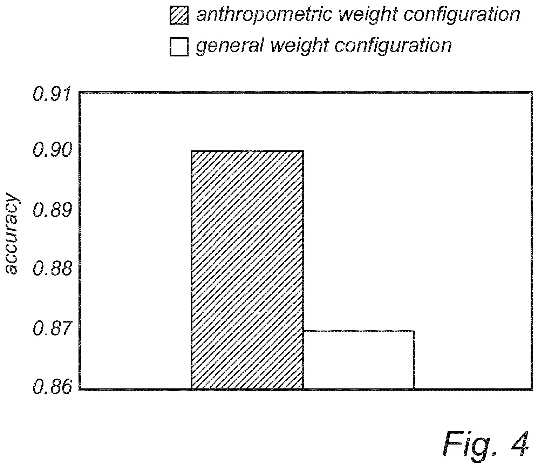

Fig. 4 illustrates the mean accuracy yielded for the general configuration as well as from the anthropometric configuration. In average, the general configuration reaches stress prediction accuracies of 87.03% while the anthropometric configuration reaches an average accuracy of 90%.

These results show that selecting specific weight configurations based on anthropometric features can increase the prediction capabilities of self-reported stress levels.

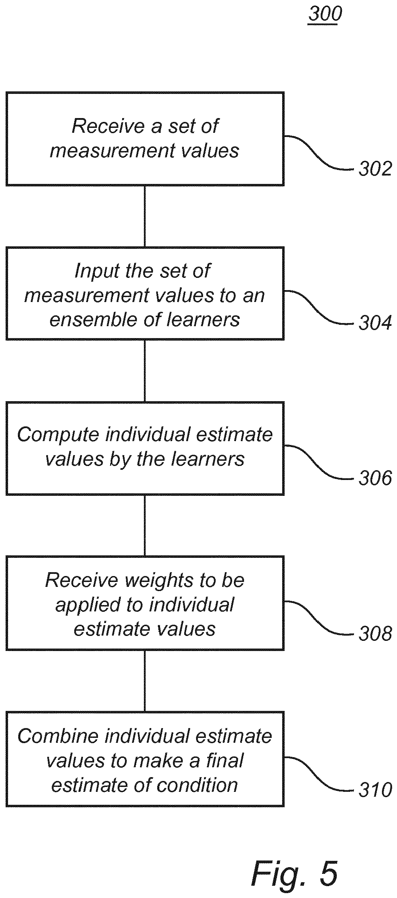

Referring now to Fig. 5, a method 300 for estimating a condition of a person will be generally described.

The method comprises receiving, step 302, a set of measurement values comprising a plurality of physiological measurement values and a plurality of context measurement values.

The received set of measurement values is input, step 304, to trained machine learning module comprising an ensemble of learners. The learners which are relevant for estimating the condition of the person may be selected based e.g. on input of relevance of learners for the specific person.

Also, or alternatively, the trained machine learning module may detect that no measurement values are received from a sensor. If so, learners that are trained to make an estimate of the condition of the person based on features extracted from measurements by the sensor may be disregarded. For instance, the learner may not be addressed such that no estimate is computed by the learner.

The learners, for instance each selected learner, may then compute, step 306, individual estimate values of the condition of the person based on the input measurement values.

The individual estimate values may be transferred to a combiner module. The combiner module may further receive, step 308, weights to be applied to the individual estimate values. The weights may be at least partly adapted to individual characteristics, such as anthropometric features, of the person for which a condition is estimated. The weights may also be adapted to a context the person is in.

The combiner module may then combine, step 310, the individual estimate values based on the received weights in order to make a final estimate of the condition of the person.

In the above the invention has mainly been described with reference to a limited number of embodiments. However, as is readily appreciated by a person skilled in the art, other embodiments than the ones disclosed above are equally possible within the scope of the invention, as defined by the appended claims.

## Claims

1. A computer-implemented method for estimating a condition of a person (102), said method comprising:
receiving (302) a set of measurement values, said set of measurement values comprising a plurality of physiological measurement values applying to the condition of the person (102) and a plurality of context measurement values, wherein said set of measurement values are obtained by a set of sensors (202a-d) comprising a plurality of physiological sensors (202a-b) and at least one environment sensor (202c-d), wherein each physiological measurement value is obtained by a physiological sensor (202a-b) making a measurement on the person (102) and wherein each context measurement value is obtained by an environment sensor (202c-d) making a measurement on an environment in which the person (102) is located;
inputting (304) the set of measurement values to an ensemble of learners (110) for estimating the condition of the person (102), wherein each learner (110) in the ensemble of learners (110) is trained to make an estimate of the condition of the person (102) based exclusively on features which are extracted from measurements by a single physiological sensor (202a-b) or environment sensor (202c-d) and wherein each learner (110), depending on availability amongst the received set of measurement values, receives measurement values relevant to the features as input;
computing (306), by a learner (110) in the ensemble of learners (110) for which measurement values corresponding to the features used by the learner (110) is available, an individual estimate value of the condition of the person (102), wherein said computing is performed by a plurality of learners (110) to determine a plurality of individual estimate values;
receiving (308) weights to be applied to the individual estimate values determined by the learners (110) in the ensemble of learners (110), wherein the weights are at least partly adapted to individual characteristics of the person (102); and
combining (310) individual estimate values based on the received weights to make a final estimate of the condition of the person (102).

2. The method according to claim 1, wherein the weights are at least partly based on a personal dataset for the person (102) whose condition is to be estimated, the personal dataset relating measurement values obtained for the person (102) to levels of the condition.

3. The method according to claim 1, wherein the weights are at least partly based on a database (114) storing information about weights in relation to characteristics of persons.

4. The method according to any one of the preceding claims, further comprising selecting learners (110) that are relevant for estimating the condition for the person (102), wherein said computing is performed by the selected learners (110).

5. The method according to any one of the preceding claims, further comprising detecting that no measurement values are received from a sensor among the set of sensors; and disregarding learners (110) among the ensemble of learners that are trained to make an estimate of the condition of the person (102) based on features which are extracted from measurements by the sensor from which no measurement values are received.

6. The method according to any one of the preceding claims, wherein the plurality of context measurement values in the set of measurement values are inputted to a weight selector for determining weights, wherein the weights are at least partly adapted to the environment in which the person (102) is located.

7. The method according to any one of the preceding claims, further comprising: determining a number of learners (110) which are to contribute to a final estimate of the condition of the person (102); and adapting the received weights based at least on the determined number of learners (110) for which individual estimate values are to contribute to the final prediction value.

8. The method according to any one of the preceding claims, wherein said condition is a stress level of the person (102).

9. The method according to claim 8, wherein said set of measurement values comprises at least one in the group of:
physiological measurement values from an electrocardiogram, ECG, sensor;
physiological measurement values from a galvanic skin response, GSR, sensor;
context measurement values from a light level sensor;
context measurement values from a positioning sensor.

10. A computer program product comprising a computer-readable medium with computer-readable instructions such that when executed on a processing unit the computer program product will cause the processing unit to perform the method according to any one of claims 1-9.

11. A device (100) for estimating a condition of a person (102), said device (102) comprising:
a data input module (104), which is configured to receive a set of measurement values, said set of measurement values comprising a plurality of physiological measurement values applying to the condition of the person (102) and a plurality of context measurement values, wherein said set of measurement values are obtained by a set of sensors (202a-d) comprising a plurality of physiological sensors (202a-b) and a plurality of environment sensors (202c-d), wherein each physiological measurement value is obtained by a physiological sensor (202a-b) making a measurement on the person (102) and wherein each context measurement value is obtained by an environment sensor (202c-d) making a measurement on an environment in which the person (102) is located;
a trained machine learning module (106), which comprises an ensemble of learners (110) for estimating the condition of the person (102), wherein each learner (110) in the ensemble of learners (110) is trained to make an estimate of the condition of the person (102) based exclusively on features which are extracted from measurements by a single physiological sensor (202a-b) or environment sensor (202c-d), wherein said trained machine learning module (106) is configured to receive the set of measurement values from the data input module (104) and wherein each learner (110), depending on availability amongst the received set of measurement values, receives measurement values relevant to the features as input;
said trained machine learning module (106) being further configured to compute, by a learner (110) in the ensemble of learners for which measurement values corresponding to the features used by the learner (110) is available, an individual estimate value of the condition of the person (102), wherein said computing is performed by a plurality of learners (110) to determine a plurality of individual estimate values; and
a combiner module (108), which is configured to receive weights to be applied to the individual estimate values determined by the learners (110) in the ensemble of learners, wherein the weights are at least partly adapted to individual characteristics of the person (102); and
wherein the combiner module (108) is further configured to combine individual estimate values based on the received weights to make a final estimate of the condition of the person (102).

## Patentansprüche

1. Computerumgesetztes Verfahren zur Beurteilung eines Zustands einer Person (102), wobei das Verfahren folgende Schritte umfasst:
Empfangen (302) einer Menge von Messwerten, wobei die Menge von Messwerten eine Vielzahl von physiologischen Messwerten, die den Zustand der Person (102) betreffen, und eine Vielzahl von Kontextmesswerten umfasst, wobei die Menge von Messwerten durch eine Menge von Sensoren (202a-d) erzielt wird, die eine Vielzahl von physiologischen Sensoren (202ab) und mindestens einen Umgebungssensor (202c-d) umfasst, wobei jeder physiologische Messwert dadurch erzielt wird, dass ein physiologischer Sensor (202a-b) eine Messung an der Person (102) vornimmt, und wobei jeder Kontextmesswert dadurch erzielt wird, dass ein Umgebungssensor (202c-d) eine Messung an einer Umgebung, in der sich die Person (102) befindet, vornimmt;
Eingeben (304) der Menge von Messwerten in eine Gesamtheit von Lernvorrichtungen (110) zum Beurteilen des Zustands der Person (102), wobei jede Lernvorrichtung (110) in der Gesamtheit von Lernvorrichtungen (110) eingelernt wird, um eine Beurteilung des Zustands der Person (102) basierend ausschließlich auf Merkmalen, die aus Messungen von einem einzigen physiologischen Sensor (202a-b) oder Umgebungssensor (202c-d) entnommen werden, vorzunehmen, und wobei jede Lernvorrichtung (110) in Abhängigkeit von der Verfügbarkeit in der empfangenen Menge von Messwerten Messwerte, die für die Merkmale als relevant sind, als Eingabe empfängt;
Berechnen (306), durch eine Lernvorrichtung (110) in der Gesamtheit von Lernvorrichtungen (110), für welche Messwerte, die den Merkmalen entsprechen, die von der Lernvorrichtung (110) verwendet werden, verfügbar sind, eines individuellen Beurteilungswertes des Zustands der Person (102), wobei das Berechnen von einer Vielzahl von Lernvorrichtungen (110) ausgeführt wird, um eine Vielzahl von individuellen Beurteilungswerten zu bestimmen;
Empfangen (308) von Gewichtungen, die auf die individuellen Beurteilungswerte, die von den Lernvorrichtungen (110) in der Gesamtheit von Lernvorrichtungen (110) bestimmt werden, anzuwenden sind, wobei die Gewichtungen mindestens teilweise an individuelle Kennzeichen der Person (102) angepasst sind; und
Kombinieren (310) von individuellen Beurteilungswerten basierend auf den empfangenen Gewichtungen, um eine endgültige Beurteilung des Zustands der Person (102) vorzunehmen.

2. Verfahren nach Anspruch 1, wobei die Gewichtungen mindestens teilweise auf einem persönlichen Datensatz für die Person (102), deren Zustand zu beurteilen ist, basieren, wobei der persönliche Datensatz Messwerte, die für die Person (102) erzielt werden, mit Niveaus des Zustands verknüpft.

3. Verfahren nach Anspruch 1, wobei die Gewichtungen mindestens teilweise auf einer Datenbank (114) basieren, die Informationen über Gewichtungen mit Bezug auf Kennzeichen von Personen speichert.

4. Verfahren nach einem der vorhergehenden Ansprüche, ferner umfassend das Auswählen von Lernvorrichtungen (110), die für das Beurteilen des Zustands für die Person (102) relevant sind, wobei das Berechnen durch die ausgewählten Lernvorrichtungen (110) erfolgt.

5. Verfahren nach einem der vorhergehenden Ansprüche, ferner umfassend das Detektieren, dass von einem Sensor aus der Menge von Sensoren keine Messwerte empfangen werden; und das Ignorieren von Lernvorrichtungen (110) aus der Gesamtheit von Lernvorrichtungen, die eingelernt sind, um eine Beurteilung des Zustands der Person (102) basierend auf Merkmalen vorzunehmen, die aus Messungen von dem Sensor, von dem keine Messwerte empfangen werden, entnommen werden.

6. Verfahren nach einem der vorhergehenden Ansprüche, wobei die Vielzahl von Kontextmesswerten in der Menge von Messwerten in eine Gewichtungsauswahlvorrichtung eingegeben wird, um Gewichtungen zu bestimmen, wobei die Gewichtungen mindestens teilweise an die Umgebung, in der sich die Person (102) befindet, angepasst werden.

7. Verfahren nach einem der vorhergehenden Ansprüche, ferner umfassend: das Bestimmen einer Anzahl von Lernvorrichtungen (110), die zu einer endgültigen Beurteilung des Zustands der Person (102) beitragen sollen; und das Anpassen der empfangenen Gewichtungen basierend mindestens auf der bestimmten Anzahl von Lernvorrichtungen (110), für welche individuelle Beurteilungswerte zum endgültigen Vorhersagewert beitragen sollen.

8. Verfahren nach einem der vorhergehenden Ansprüche, wobei der Zustand ein Stressniveau der Person (102) ist.

9. Verfahren nach Anspruch 8, wobei die Menge von Messwerten mindestens einen aus der Gruppe umfasst von:
physiologischen Messwerten von einem Elektrokardiogramm-, ECG, Sensor;
physiologischen Messwerten von einem galvanischen Hautreaktions-, GSR, Sensor;
Kontextmesswerten von einem Lichtniveausensor;
Kontextmesswerten von einem Positionsbestimmungssensor.

10. Computerprogrammprodukt, umfassend einen computerlesbaren Datenträger mit computerlesbaren Anweisungen, so dass das Computerprogrammprodukt, wenn es auf einer Verarbeitungseinheit ausgeführt wird, bewirkt, dass die Verarbeitungseinheit das Verfahren nach einem der Ansprüche 1 bis 9 ausführt.

11. Vorrichtung (100) zum Beurteilen eines Zustands einer Person (102), wobei die Vorrichtung (102) umfasst:
ein Dateneingabemodul (104), das konfiguriert ist, um eine Menge von Messwerten zu empfangen, wobei die Menge von Messwerten eine Vielzahl von physiologischen Messwerten, die den Zustand der Person (102) betreffen, und eine Vielzahl von Kontextmesswerten umfasst, wobei die Menge von Messwerten von einer Menge von Sensoren (202a-d), die eine Vielzahl von physiologischen Sensoren (202a-b) und eine Vielzahl von Umgebungssensoren (202c-d) umfasst, erzielt wird, wobei jeder physiologische Messwert durch einen physiologischen Sensor (202a-b), der eine Messung an der Person (102) vornimmt, erzielt wird, und wobei jeder Kontextmesswert durch einen Umgebungssensor (202c-d), der eine Messung an einer Umgebung, in der sich die Person (102) befindet, vornimmt, erzielt wird;
ein eingelerntes Maschinenlernmodul (106), das eine Gesamtheit von Lernvorrichtungen (110) zum Beurteilen des Zustands der Person (102) umfasst, wobei jede Lernvorrichtung (110) in der Gesamtheit von Lernvorrichtungen (110) eingelernt ist, um eine Beurteilung des Zustands der Person (102) basierend ausschließlich auf Merkmalen, die aus Messungen von einem einzigen physiologischen Sensor (202a-b) oder Umgebungssensor (202c-d) entnommen werden, vorzunehmen, wobei das eingelernte Maschinenlernmodul (106) konfiguriert ist, um die Menge von Messwerten von dem Dateneingabemodul (104) zu empfangen, und wobei jede Lernvorrichtung (110) in Abhängigkeit von der Verfügbarkeit in der empfangenen Menge von Messwerten Messwerte, die für die Merkmale relevant sind, als Eingabe empfängt;
wobei das eingelernte Maschinenlernmodul (106) ferner konfiguriert ist, um durch eine Lernvorrichtung (110) in der Gesamtheit von Lernvorrichtungen, für welche Messwerte, die den Merkmalen, die von der Lernvorrichtung (110) verwendet werden, entsprechen, verfügbar sind, einen individuellen Beurteilungswert des Zustands der Person (102) zu berechnen, wobei das Berechnen durch eine Vielzahl von Lernvorrichtungen (110) erfolgt, um eine Vielzahl von individuellen Beurteilungswerten zu bestimmen; und
ein Kombinationsmodul (108), das konfiguriert ist, um Gewichtungen zu empfangen, die auf die individuellen Beurteilungswerte anzuwenden sind, die von den Lernvorrichtungen (110) in der Gesamtheit von Lernvorrichtungen bestimmt werden, wobei die Gewichtungen mindestens teilweise an individuelle Kennzeichen der Person (102) angepasst sind; und
wobei das Kombinationsmodul (108) ferner konfiguriert ist, um individuelle Beurteilungswerte basierend auf den empfangenen Gewichtungen zu kombinieren, um eine endgültige Beurteilung des Zustands der Person (102) vorzunehmen.

## Revendications

1. Procédé mis en œuvre par informatique pour estimer un état d'une personne (102), ledit procédé comprenant :
la réception (302) d'un ensemble de valeurs de mesure, ledit ensemble de valeurs de mesure comprenant une pluralité de valeurs de mesure physiologiques s'appliquant à l'état de la personne (102) et une pluralité de valeurs de mesure de contexte, dans lequel ledit ensemble de valeurs de mesures sont obtenues par un ensemble de capteurs (202a-d) comprenant une pluralité de capteurs physiologiques (202a-b) et au moins un capteur de l'environnement (202c-d), dans lequel chaque valeur de mesure physiologique est obtenue par un capteur physiologique (202a-b) effectuant une mesure de la personne (102) et dans lequel chaque valeur de mesure de contexte est obtenue par un capteur d'environnement (202c-d) effectuant une mesure d'un environnement dans lequel la personne (102) se situe ;
l'entrée (304) de l'ensemble des valeurs de mesure dans un ensemble d'apprenants (110) pour estimer l'état de la personne (102), dans lequel chaque apprenant (110) de l'ensemble d'apprenants (110) est entraîné pour effectuer une estimation de l'état de la personne (102) sur la base exclusivement de caractéristiques qui sont extraites de mesures par un seul capteur physiologique (202a-b) ou un capteur d'environnement (202c-d) et dans lequel chaque apprenant (110), en fonction de la disponibilité au sein de l'ensemble reçu de valeurs de mesure, reçoit des valeurs de mesure pertinentes pour les caractéristiques en tant qu'entrée ;
le calcul (306), par un apprenant (110) dans l'ensemble d'apprenants (110) pour lequel des valeurs de mesure correspondant aux caractéristiques utilisées par l'apprenant (110) sont disponibles, d'une valeur d'estimation individuelle de l'état de la personne (102), dans lequel ledit calcul est effectué par une pluralité d'apprenants (110) pour déterminer une pluralité de valeurs d'estimation individuelles ;
la réception (308) de poids à appliquer aux valeurs d'estimation individuelles déterminées par les apprenants (110) dans l'ensemble d'apprenants (110), dans lequel les poids sont au moins partiellement adaptés à des caractéristiques individuelles de la personne (102) ; et
la combinaison (310) de valeurs d'estimation individuelles sur la base des poids reçus pour effectuer une estimation finale de l'état de la personne (102).

2. Procédé selon la revendication 1, dans lequel les poids sont au moins partiellement basés sur un jeu de données personnelles pour la personne (102) dont l'état doit être estimé, le jeu de données personnelles liant des valeurs de mesure obtenues pour la personne (102) à des niveaux de l'état.

3. Procédé selon la revendication 1, dans lequel les poids sont au moins partiellement basés sur une base de données (114) stockant de l'information sur des poids en lien avec des caractéristiques de personnes.

4. Procédé selon l'une quelconque des revendications précédentes, comprenant en outre la sélection d'apprenants (110) pertinents pour estimer l'état de la personne (102), dans lequel ledit calcul est effectué par les apprenants sélectionnés (110).

5. Procédé selon l'une quelconque des revendications précédentes, comprenant en outre la détection qu'aucune des valeurs de mesure n'est reçue d'un capteur parmi l'ensemble de capteurs ; et la non prise en compte d'apprenants (110) parmi l'ensemble d'apprenants qui sont entraînés pour effectuer une estimation de l'état de la personne (102) sur la base de caractéristiques qui sont extraites de mesures par le capteur de la part duquel aucune valeur de mesure n'a été reçue.

6. Procédé selon l'une quelconque des revendications précédentes, dans lequel la pluralité des valeurs de mesure de contexte dans l'ensemble de valeurs de mesure sont entrées dans un sélecteur de poids pour déterminer des poids, dans lequel les poids sont au moins partiellement adaptés à l'environnement dans lequel la personne (102) se situe.

7. Procédé selon l'une quelconque des revendications précédentes, comprenant en outre : la détermination d'un certain nombre d'apprenants (110) qui doivent contribuer à une estimation finale de l'état de la personne (102) ; et l'adaptation des poids reçus sur la base au moins du nombre d'apprenants (110) déterminé pour lesquels des valeurs d'estimation individuelles doivent contribuer à la valeur de prédiction finale.

8. Procédé selon l'une quelconque des revendications précédentes, dans lequel ledit état est un niveau de stress de la personne (102).

9. Procédé selon la revendication 8, dans lequel ledit ensemble de valeurs de mesure comprend au moins les unes parmi le groupe suivant :
des valeurs de mesure physiologiques d'un capteur pour électrocardiogramme, ECG ;
des valeurs de mesure physiologiques d'un capteur de réaction cutanée galvanique, GSR ;
des valeurs de mesure de contexte d'un capteur de niveau de lumière ;
des valeurs de mesure de contexte d'un capteur de positionnement.

10. Produit de programme informatique comprenant un support lisible par ordinateur avec des instructions lisibles par ordinateur de sorte que, lorsqu'il est exécuté par une unité de traitement, le produit de programme informatique va faire en sorte que l'unité de traitement mette en oeuvre le procédé selon l'une quelconque des revendications 1-9.

11. Dispositif (100) pour estimer un état d'une personne (102), ledit dispositif (102) comprenant :
un module d'entrée de données (104) qui est configuré pour recevoir un ensemble de valeurs de mesure, ledit ensemble de valeurs de mesure comprenant une pluralité de valeurs de mesure physiologiques s'appliquant à l'état de la personne (102) et une pluralité de valeurs de mesure de contexte, dans lequel ledit ensemble de valeurs de mesure sont obtenues par un ensemble de capteurs (202a-d) comprenant une pluralité de capteurs physiologiques (202a-b) et une pluralité de capteur d'environnement (202c-d), dans lequel chaque valeur de mesure physiologique est obtenue par un capteur physiologique (202a-b) effectuant une mesure de la personne (102) et dans lequel chaque valeur de mesure de contexte est obtenue par un capteur d'environnement (202c-d) effectuant une mesure d'un environnement dans lequel la personne (102) se situe ;
un module d'apprentissage automatique entraîné (106) qui comprend un ensemble d'apprenants (110) pour estimer l'état de la personne (102), dans lequel chaque apprenant (110) dans l'ensemble d'apprenants (110) est entraîné pour effectuer une estimation de l'état de la personne (102) sur la base exclusivement de caractéristiques qui sont extraites de mesures par un seul capteur physiologique (202a-b) ou un capteur d'environnement (202c-d), dans lequel ledit module d'apprentissage automatique entraîné (106) est configuré pour recevoir l'ensemble de valeurs de mesure du module d'entrée de données (104) et dans lequel chaque apprenant (110), en fonction d'une disponibilité au sein de l'ensemble de valeurs de mesure reçues, reçoit des valeurs de mesure pertinentes pour les caractéristiques en tant qu'entrée ;
ledit module d'apprentissage automatique entraîné (106) étant en outre configuré pour calculer, grâce à un apprenant (110) dans l'ensemble d'apprenants pour lequel des valeurs de mesure correspondant aux caractéristiques utilisées par l'apprenant (110) sont disponibles, une valeur d'estimation individuelle de l'état de la personne (102), dans lequel ledit calcul est réalisé par une pluralité d'apprenants (110) pour déterminer une pluralité de valeurs d'estimation individuelles ; et
un module de combinaison (108) configuré pour recevoir des poids à appliquer aux valeurs d'estimation individuelles déterminées par les apprenants (110) dans l'ensemble d'apprenants, dans lequel les poids sont au moins partiellement adaptés à des caractéristiques individuelles de la personne (102) ; et
dans lequel le module de combinaison(108) est en outre configuré pour combiner des valeurs d'estimation individuelles sur la base des poids reçus pour effectuer une estimation finale de l'état de la personne (102).
